**Europäisches Patentamt**

**European Patent Office**

**Office européen des brevets**

(19)

(11) Veröffentlichungsnummer: **0 070 538**
A1

(12)

# EUROPÄISCHE PATENTANMELDUNG

(21) Anmeldenummer: **82106413.6**

(22) Anmeldetag: **16.07.82**

(51) Int. Cl.³: **A 61 F 13/08,** A 61 F 5/14

(30) Priorität: **22.07.81 DE 3128908**

(43) Veröffentlichungstag der Anmeldung: **26.01.83**
**Patentblatt 83/4**

(84) Benannte Vertragsstaaten: **AT BE CH FR GB IT LI LU NL SE**

(71) Anmelder: **Hildebrandt, Hans-Dietrich, Dr. med., Hohlesteinweg 16, D-3501 Ahnatal (DE)**

(72) Erfinder: **Hildebrandt, Hans-Dietrich, Dr. med., Hohlesteinweg 16, D-3501 Ahnatal (DE)**

(74) Vertreter: **Freiherr von Schorlemer, Reinfried, Brüder-Grimm-Platz 4, D-3500 Kassel (DE)**

(54) **Elastische Knöchelstütze.**

(57) Eine elastische Knöchelstütze (1) ist mit einem an das Fussrelief angepassten Pronationskeil (2) versehen. Der Pronationskeil ist entweder aussen oder innen an die Knöchelstütze angeklebt oder in eine an die Knöchelstütze angearbeitete Tasche eingelegt.

EP 0 070 538 A1

ACTORUM AG

Elastische Knöchelstütze

Elastische Knöchelstützen dienen zur äußeren Stabilisierung von Bandinsuffizienzen des lateralen Bandapparates des oberen Sprunggelenks des menschlichen Fußes. Insuffizienzen dieser Art entstehen beispielsweise durch Unfälle, insbesondere auch Sportunfälle, oder sind anlagebedingt im Rahmen einer Bindegewebsschwäche (Hypermobilität).

Da elastische Knöchelstützen dieser Art häufig nicht zum Ausgleich der vorhandenen Instabilität im Sprunggelenk ausreichen, die Unsicherheiten, verstärkte Umknickneigung und Schmerzen zur Folge hat, wird vielfach zusätzlich ein Pronationskeil verordnet. Ein derartiger Pronationskeil besteht aus einem keilförmigen, aus Leder oder Gummi hergestellten Klotz, der unter dem Schuhsohlenrand angebracht wird und durch Anheben des äußeren Fußrandes ein Umknicken des Fußes beim Gehen oder Laufen verhindern soll. Da der Pronationskeil am Schuh fest verankert ist und außerdem an jedem in Frage kommenden Schuh vorgesehen sein sollte, ist die Verordnung eines Pronationskeils sehr kostenintensiv.

Entsprechende Pronationskeile werden auch verordnet, wenn Schäden im Bereich des medialen Kniegelenkkompartimentes, z.B. Arthrose oder Meniscusschäden, ohne operativen Eingriff behandelt werden sollen.

Bekannt ist es auch bereits, den Pronationskeil nicht an der Schuhsohle zu befestigen, sondern unter einer Einlegesohle anzuordnen. Diese Maßnahme wird jedoch nur dann vorgesehen, wenn zusätzliche Fußfehler vorhanden sind und aus diesem Grunde eine Einlegesohle ohnehin verordnet werden muß. Die Benutzung einer lediglich aus Gründen des Pronationskeils verordneten Einlegesohle wäre aus orthopädisch medizinischer Sicht unsinnig, da ein gesunder, muskelkräftiger Fuß durch die passive Einlagenstützung geschwächt würde.

0070538

Der Erfindung liegt die Aufgabe zugrunde, hier Abhilfe zu schaffen und eine preisgünstige Möglichkeit zur vielfachen Ausnutzung der Vorteile des Pronationskeils vorzuschlagen.

Zur Lösung dieser Aufgabe sind die kennzeichnenden Merkmale des Anspruchs 1 vorgesehen.

Die Erfindung geht von der Erkenntnis aus, daß die Kombination einer elastischen Knöchelstütze mit einem Pronationskeil ein ideales Mittel zur Behandlung der beschriebenen Insuffizienzen darstellt. Denn einerseits wird die Wirkung des Pronationskeils durch die Stützwirkung der elastischen Knöchelstütze unterstützt, und andererseits ist die erfindungsgemäße Knöchelstütze völlig unabhängig von dem im Einzelfall getragenen Schuhwerk, da sie zusammen mit jedem beliebigen Schuh und sogar auch ohne Schuh getragen werden kann. Dadurch ist nicht nur die Verordnung eines Pronationskeils mit beträchtlich geringeren Kosten als bisher verbunden, sondern beispielsweise auch für Sporttreibende die Möglichkeit gegeben, sich auf eigene Initiative und zu geringen Kosten vorbeugend mit einem Pronationskeil zu versehen. Abgesehen davon bringt die Erfindung den besonderen Vorteil mit sich, daß der Pronationskeil der Fußform angepaßt ist und unmittelbar auf den Fuß einwirkt. Dadurch ergibt sich eine viel stärkere Wirkung als bei der nur mittelbaren, über den Schuh oder die Einlegesohle erfolgenden Einwirkung nach bekanntem Vorbild.

Weitere Vorteile der Erfindung sind in den Unteransprüchen gekennzeichnet.

Die Erfindung wird nachfolgend in Verbindung mit der beiliegenden Zeichnung an zwei Ausführungsbeispielen näher erläutert. Es zeigen:

Fig. 1 bis 3 schematisch eine erfindungsgemäße elastische Knöchelstütze mit integriertem Pronationskeil von der Seite, von hinten und von unten; und

Fig. 4 eine vergrößerte Seitenansicht des Pronationskeils.

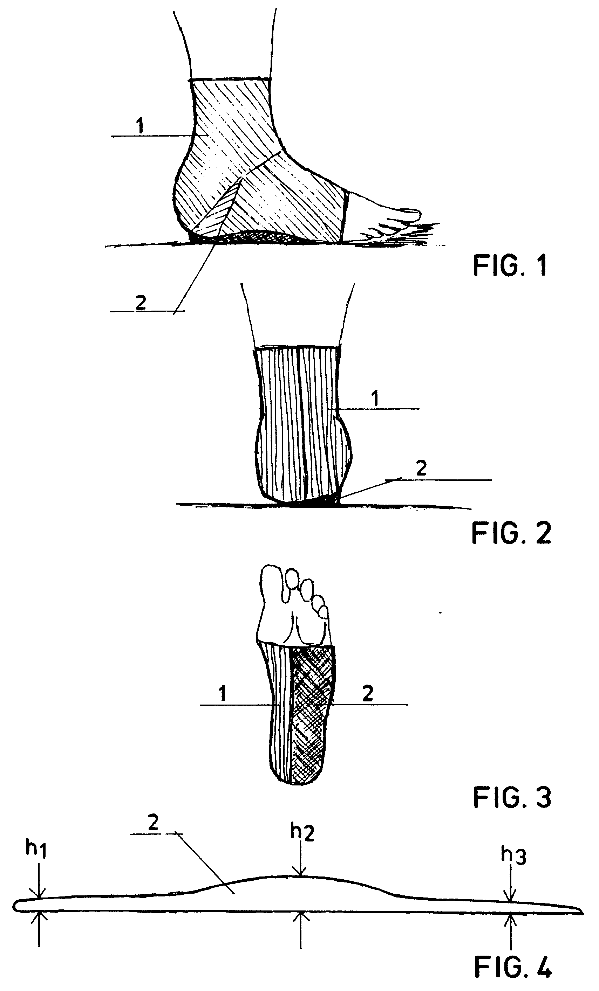

Gemäß Fig. 1 bis 3 ist in eine übliche elastische Knöchelstütze ein Pronationskeil 2 integriert. Wie insbesondere
Fig. 3 zeigt, ist die Breite des für den rechten Fuß verwendbaren Pronationskeils etwa gleich der Hälfte der Bußbreite. In Längsrichtung des Fußes erstreckt sich der Pronationskeil etwa von der Ferse bis zur Basis des/ fünften Mittelfußknochens. Der Pronationskeil 2 ist außerdem der Fußform, insbesondere der
Form der Fußauftrittsfläche angepaßt. Im Bereich der Fußauftrittsfläche und im Bereich der Basis des fünften Mittelfußknochens ist der Pronationskeil 2 weniger hoch als im
Bereich des Außenknöchels und daher im Gegensatz zu einem
an einer Schuh- oder Einlegesohle befestigten geraden Keil
etwa in der aus Fig. 4 ersichtlichen Weise gebogen. Von
außen nach innen nimmt die Höhe des Pronationskeils 2, wie
in Fig. 2 angedeutet ist, keilförmig allmählich ab.

Besonders günstige Verhältnisse ergeben sich, wenn der
Pronationskeil 2 im Bereich der Auftrittsfläche des Fersenbeins eine durchschnittliche Höhe $h_1$ von etwa vier Millimeter, im Bereich des Außenknöchels bzw. distal davon eine
Höhe $h_2$ von etwa sieben bis acht Millimetern und im Bereich
der Basis des fünften Mittelfußknochens eine durchschnittliche Höhe $h_3$ von etwa drei Millimetern und daher von der
Seite her gesehen, eine lanzettenförmige Gestalt aufweist.
Um eine bessere Anpassung an unterschiedliche Fußgrößen und Fußformen zu erzielen, wird die erfindungsgemäße Knöchelstütze zweckmäßig in mehreren Varianten hergestellt und verordnet, die jeweils entsprechend einer
möglichst großen Anzahl gleichartiger oder gleich großer
Standardfußformen bemessen sind. Auch das allmähliche
Auslaufen des Pronationskeils 2 und dessen Länge sind
entsprechend den durchschnittlichen Fußgrößen und Fußformen zu bemessen.

Der Pronationskeil 2 besteht vorzugsweise aus Leder,
Korkleder oder einem etwas nachgiebigen Kunststoff.

Die Befestigung des Pronationskeils 2 an der Knöchelstütze 1
erfolgt entweder von innen oder, wie in der Zeichnung dargestellt ist, von außen durch Kleben od. dgl. Möglich ist es
auch, in die Knöchelstütze eine der Größe des Pronationskeils 2 entsprechende Tasche einzuarbeiten, in die der Pronationskeil eingeschoben wird. Auf diese Weise kann die
Knöchelstütze 1 wahlweise mit oder ohne Pronationskeil 2
angewendet werden. Gemäß einer weiteren Ausführungsform
kann der Pronationskeil auch mit einem dünnen Stoff taschenförmig umnäht  bzw. an die Knöchelstütze 1 angenäht werden.

Der in der Zeichnung dargestellte Pronationskeil 2 ist nur
für den rechten Fuß geeignet. Für den linken Fuß ist ein
entsprechender Pronationskeil mit entgegengesetzter Keilform
vorzusehen.

# 0070538

Patentanwalt
Diplom-Physiker
**Reinfried Frhr. v. Schorlemer**


D-3500 Kassel
Brüder-Grimm-Platz 4
Telefon (0561) 15335

D 5151

Dr. Hans-Dietrich Hildebrandt, 3501 Ahnatal

Ansprüche

1) Elastische Knöchelstütze, dadurch gekennzeichnet, daß sie mit einem an das Fußrelief angepaßten Pronationskeil (2) versehen ist.

2) Knöchelstütze nach Anspruch 1, dadurch gekennzeichnet, daß der Pronationskeil (2) außen oder innen an die Knöchelstütze (1) angeklebt ist.

3) Knöchelstütze nach Anspruch 1, dadurch gekennzeichnet, daß der Pronationskeil (2) in eine an die Knöchelstütze (1) angearbeitete Tasche eingelegt ist.

4) Knöchelstütze nach einem der Ansprüche 1 bis 3, dadurch gekennzeichnet, daß die Breite des Pronationskeils (2) etwa der halben Breite der Fußauftrittsfläche entspricht.

5) Knöchelstütze nach einem der Ansprüche 1 bis 4, dadurch gekennzeichnet, daß die Höhe des Pronationskeils (2) im Bereich der Auftrittsfläche des Fersenbeins etwa vier Millimeter, im Bereich des Außenknöchels bzw. distal davon etwa sieben bis acht Millimeter und im Bereich der Basis des Mittelfußknochens etwa drei Millimeter beträgt.

0070538

1/1

FIG. 1

FIG. 2

FIG. 3

FIG. 4

## EUROPÄISCHER RECHERCHENBERICHT

**Europäisches Patentamt**

Nummer der Anmeldung

EP 82 10 6413.6

| | EINSCHLÄGIGE DOKUMENTE | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| A | DE - C - 452 409 (MAY) <br> * Fig. 1, 2 * <br> -- | 1,3 | A 61 F 13/08 <br> A 61 F 5/14 |
| A | CH - A - 398 875 (ZUMSTEIN) <br> * Fig. * <br> -- | 1,3 | |
| A | CH - A - 246 127 (ASCHWANDEN) <br> * Fig. 1 * <br> -- | 1,2 | **RECHERCHIERTE SACHGEBIETE (Int. Cl.³)** |
| A | CH - A - 171 170 (RATHGEBER) <br> * Fig. 1 bis 5 * <br> -- | 1,3 | A 61 F 5/00 <br> A 61 F 13/00 |
| A | CH - A - 126 919 (MAY) <br> * Fig. 1, 6 * <br> -- | 1,3 | |
| A | US - A - 1 477 357 (JENSEN) <br> * Anspruch 1 ; Fig. 1 bis 3 * <br> ---- | 1,3 | |

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

| X | Der vorliegende Recherchenbericht wurde für alle Patentansprüche erstellt. |
|---|---|

| Recherchenort | Abschlußdatum der Recherche | Prüfer |
|---|---|---|
| Berlin | 29-09-1982 | KANAL |

EPA form 1503.1   06.78